(19) Europäisches Patentamt
European Patent Office
Office européen des brevets

(11) Publication number: **0 120 583**
**B1**

(12) **EUROPEAN PATENT SPECIFICATION**

(45) Date of publication of patent specification: 12.08.87

(51) Int. Cl.[4]: **A 61 K 49/04**, C 07 C 43/12

(21) Application number: 84301009.1

(22) Date of filing: 16.02.84

(54) **Perfluorobromoalkyl ethers for use as X-ray contrast agents.**

(30) Priority: 28.02.83 US 470343

(43) Date of publication of application:
03.10.84 Bulletin 84/40

(45) Publication of the grant of the patent:
12.08.87 Bulletin 87/33

(84) Designated Contracting States:
FR GB

(56) References cited:
US-A-3 975 512

CHEMICAL ABSTRACTS, vol. 98, Chemical Substance Index, 30th June 1983, page 1470CS, Chemical Abstracts Service, Columbus, Ohio, US;

(73) Proprietor: ADAMANTECH, INC.
Second and Green Streets P.O. Box 1195
Marcus Hook Pennsylvania 19061 (US)

(73) Proprietor: CHILDREN'S HOSPITAL MEDICAL CENTER
Elland and Bethesda Avenues
Cincinnati Ohio 45229 (US)

(72) Inventor: Tamborski, Christ
4404 Stonecastle Drive
Dayton, OH 45440 (US)
Inventor: Clark, Leland C., Jr.
218 Greendale Avenue
Cincinnati, OH 45220 (US)

(74) Representative: Lewin, John Harvey et al
Elkington and Fife High Holborn House 52/54
High Holborn
London WC1V 6SH (GB)

EP 0 120 583 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to perfluorobromoalkyl ethers for use as X-ray radiopaque agents particularly in connection with radiation contrast imaging in animals, including humans. Radiopaque materials are useful, for example, in quantitating coronary artery occlusive disease, in stroke, and in other occlusive diseases.

Use of brominated fluorocarbons, such as perfluorooctyl bromide, as radiopaque materials has been reported in the article by D. M. Long, et al. entitled "Radiopaque Applications of Brominated Fluorocarbon Compounds in Experimental Animals and Human Subjects" appearing in *Biochemistry Involving Carbon-Fluorine Bonds*, edited by Robert Filler, ACS Symposium Series No. 28, 1976, pages 171—189.

US—A—3,778,381 (Rosano et al) and US—A—3,453,333 (Litt et al) describe perfluorohaloalkyl ethers related to the ether compounds of this invention.

In accordance with the present invention it has been found that perfluorobromoalkyl ethers, as represented by perfluoro-1-bromobutyl-isopropyl ether, are excellent radiopaque materials. They are used in a method of rendering an internal region of an animal opaque to X-rays, which comprises introducing the radiopaque agent into the region. The ethers are stable to light and heat and are X-ray opaque. The ethers can be used neat or in aqueous dispersions, are relatively non-toxic, and transpire from mice at a good rate. In general, perfluoro chemicals having an ether linkage tend to be retained for very long periods in the reticuloendothelial system ("RES") of animals. The perfluorobromoalkyl ethers, however, have been found to transpire rapidly through the lungs, indicating that they are not retained in tissues as are other ethers.

The perfluorobromoalkyl ethers are represented by the formula (I):

$$C_mF_{2m+1}\ O\ C_nF_{2n}Br \qquad (I)$$

where n and m independently are integers of 2 to 6, preferably 2 to 4. Thus, the ethers contain from 4 to 12 carbon atoms as straight or branched-chain alkyl groups. Such ethers are RES-phobic, i.e., they will leave or will not be retained by the reticuloendothelial system of the body. Specific ethers within the scope of formula (I) include:

*F*-1-bromoethylethyl ether
*F*-1-bromoethylisopropyl ether
*F*-1-bromopropylisopropyl ether
*F*-1-bromobutylisopropyl ether
*F*-1-bromobutylethyl ether
*F*-1-bromobutylpentyl ether
*F*-1-bromohexylisopropyl ether

Generally, the ether can be prepared from an iodide precursor. For example, perfluoro-1-bromobutyl-isopropyl ether is readily prepared from perfluoro-1-iodobutylisopropyl ether by a free radical halogen exchange with bromine in the presence of ultraviolet (actinic) light as follows:

$$(CF_3)_2CFOC_4F_8I \xrightarrow{Br_{2/uv}} (CF_3)_2\ CFOC_4F_8Br$$

The foregoing reaction gives an essentially quantitative conversion of the iodide compound to the desired bromide. Preparation of various iodide ether precursors and other methods of conversion to the bromides are disclosed, for example, in US—A—3,453,333, US—A—3,470,256 and US—A—3,547,861 and in "Formation of Fluorinated Ethers in a Modified Halohydrin Reaction", Journal of Organic Chemistry, Vol. 33, No. 5, May, 1968, pages 1839—1844. Use of $(CF_3)_2\ CFOC_4F_8I$ to prepare other ethers is disclosed in US—A—3,790,607, US—A—3,781,370 and US—A—3,637,868.

The ethers will vary somewhat in their radiopacity; those ethers having higher bromine to carbon weight ratios will exhibit greater contrast. The higher bromine content ethers, having fewer carbon atoms, will be less expensive to synthesize. However, the lower carbon content ethers, such as perfluoro-1-bromoethylisopropyl ether, $(CF_3)_2CFOC_2F_4Br$, also have higher vapour pressures and therefore will transpire more rapidly from animals. Accordingly, the selection of an ether for use in a particular radiopaque application will depend upon how much contrast is required, how quickly it is desired to have the ether leave the body and, of course, how toxic the ether is to the animal. With respect to toxicity, some toxicity may be tolerable by the animal; hence, while a totally non-toxic material is preferred, a lower level of toxicity may be acceptable depending on the species of animal, the state of health of the animal, and other considerations of a pharmacological nature.

Since the perfluorobromoalkyl ethers are also good oxygen and carbon dioxide carriers, they can double as blood substitutes when suitably emulsified or dispersed in an aqueous medium or can be admixed with other perfluorocarbon blood substitutes, in the manner described in US—A—3,911,138 and US—A—4,105,798 and other blood substitute literature. Moreover, the ethers are highly miscible with or are good solvents for perfluorocarbon blood substitutes (such as those of the aforementioned patents) and

therefore can be used as diluents for other perfluorocarbon blood substitutes or as solvents for perfluorocarbon blood substitutes which are normally solids.

The ethers can be used neat or mixed with water to form aqueous dispersions such as emulsions. Water emulsions of the oil-in-water or water-in-oil type may be used. The greater the amount of perfluorobromoalkyl ether, the greater the opacity. The emulsions can contain about 10% to about 90% by volume of water, preferably 50—90% by volume, and the perfluorobromoalkyl ether may comprise as high as about 95% by volume and as low as about 5% by volume of the emulsion, preferably about 5—20% by volume. Mixtures of different ethers can be used. Normally, the emulsion will contain about 0.5—10% by weight, preferably 1—5% by weight, of an emulsifier based on the water content of the emulsion. The specific emulsifier employed is not critical, but it should itself be non-toxic or the toxicity should be tolerable, and it should form a relatively stable emulsion. Preferred emulsifiers are the non-ionic types, such as yolk phospholipid, a surfactant known to be harmless to humans. Also suitable are the polyoxyethylenes, polyoxypropylenes and copolymers thereof, available commercially under the "Pluronic" trademark, and the fluorinated surfactants described in US—A—3,828,085 and US—A—3,547,995.

Nonionic fluorine containing surfactants are particularly preferred. The fluorinated alkyl esters are one class of these surfactants, and are commercially available from 3M Company under the designations FC-93, FC-95, FC-128, FC-143, FC-430 and FC-431.

The more preferred nonionic, fluorine containing surfactants, from the standpoint of their exceptional ability to form dispersions which maintain a range of small particle size over substantial periods of time, of the order of 35 weeks to a year or more, even at room temperature, are the fluorinated amidoamine oxides described in US—A—3,828,085 (Price et al) and US—A—3,547,995 (Bartlett). These compounds may be generically described by the formula (1):

$$R_fCON(Y)—RQ \qquad (1)$$

wherein $R_f$ is a perfluoroalkyl radical of 1 to about 25 carbon atoms or a polyfluoroalkyoxyalkyl radical wherein the alkoxy group may contain 3 to about 40 carbon atoms of which at least a major portion thereof are perfluorinated and the alkyl group may contain 2 to about 40 carbon atoms, fluorinated or unfluorinated; Y is a hydrogen or alkyl of 1 to 6 carbon atoms; R is an alkylene radical of the formula:

$$—C_zH_{2z}—$$

wherein z is an integer of 1 to 6; and Q is an aliphatic amine oxide radical of the formula:

$$—N(R_5)(\rightarrow O)—R_6$$

wherein $R_5$ and $R_6$ are each alkyl radicals of 1 to 6 carbon atoms or hydroxy terminated alkyl radicals of 2 to 6 carbon atoms. In all cases the alkoxy, alkyl and alkylene groups may be straight or branched chain.

Thus, in one aspect the invention also provides an aqueous dispersion comprising a perfluorobromoalkyl ether as defined above and a fluorinated amidoamine oxide of the above formula (1).

Preferred subclasses of the surfactants of the foregoing patents are those of the following formulae (2) and (3):

$$C_nF_{2n+1}O(CF_2)_xC(=O)NH(CH_2)_yN(\rightarrow O)R^1R^2 \qquad (2)$$

wherein n is at least 3 (preferably 3—10), x is at least 2 (preferably 2—6), y is at least 1 (preferably 2—6), and $R^1$ and $R^2$ independently are alkyl radicals containing 1—6 carbon atoms;

$$C_nF_{2n+1}C(=O)NH(CH_2)_zN(\rightarrow O)R^1R^2 \qquad (3)$$

wherein n is at least 1 (preferably 3—10), z is at least 1 (preferably 2—6), and $R^1$ and $R^2$ independently are alkyl radicals containing 1 to 6 carbon atoms.

Specific amidoamine oxides within the scope of the above formula are the products described in Examples 1—6 of US—A—3,828,085, namely:

$$CF_3(CF_2)_6\overset{\overset{O}{\|}}{C}NH(CH_2)_3\overset{\overset{O}{\uparrow}}{N}(CH_3)_2$$

$$(CF_3)_2CFO(CF_2)_3\overset{\overset{O}{\|}}{C}NH(CH_2)_3\overset{\overset{O}{\uparrow}}{N}(CH_3)_2$$

$$(CF_3)_2CFO(CF_2)_5\overset{\overset{O}{\|}}{C}NH(CH_2)_3\overset{\overset{O}{\uparrow}}{N}(CH_3)_2$$

$$(CF_3)_2CFO(CF_2)_7\overset{\overset{O}{\|}}{C}NH(CH_2)_3\overset{\overset{O}{\uparrow}}{N}(CH_3)_2$$

$$(CF_3)_2CFO(CF_2)_5\overset{\overset{O}{\|}}{C}NH(CH_2)_3\overset{\overset{O}{\uparrow}}{N}(C_2H_5)_2$$

$$(CF_3)_2CFO(CF_2)_8(CH_2)_{10}\overset{\overset{O}{\|}}{C}NH(CH_2)_3\overset{\overset{O}{\uparrow}}{N}(C_2H_5)_2$$

The aqueous dispersions of the invention are prepared by any mixing technique which will provide a uniform blend of the ingredients, and preparation accordingly may be readily accomplished by the skilled formulator.

When formulating the dispersions of the invention for systemic administration, it is important not only to add electrolytes and other materials to render the dispersions physiologically acceptable (such as isotonic with mammalian cells), but also to adjust the pH, if necessary. Among the additives commonly used to render fluids physiologically acceptable are buffers such as sodium bicarbonate, and mixtures such as Ringer's Solution. Other materials conventionally employed in pharmaceutical preparations and known to the skilled formulator may also be added to the dispersions. These include viscosity modifiers, stabilizers (against degradation due to freezing or contamination, for example), anti-freeze agents, diluents, encoding agents, and the like. Among such additives may be mentioned glycerin, dimethylsulfoxide, ("DMSO"), various gelatins both natural and synthetic, and polyols such as sorbitol.

The perfluoro compound and surfactant components may be blended into water in any proportions which will provide uniform dispersions. Typical proportions are about 5 to 50% perfluoro compound based on the volume of the total composition and about 0.5 to 10% of the surfactant based on the total weight of the composition. Preferred proportions are about 10—30% by volume of the perfluoro compound and about 2—5% by weight of the surfactant, but proportions in particular cases may be varied depending on dispersibility of the PFC, particle size desired, and similar considerations.

The aqueous dispersions more usually comprise emulsions, preferably of the oil-in-water type but also including water-in-oil emulsions. In some cases the emulsions have a very fine particle size and appear transparent or solution-like to the unaided eye. The micro-emulsions which can be formulated with the dispersants of US—A—3,828,085 have this characteristic and are preferred. Colloidal suspensions, while not excluded from use in this invention, are less preferred, particularly for systemic administration, because of their larger particle size range and less stability. The above-identified blood substitute and surfactant patents provide excellent guidance to formulation of the dispersions, and attention is directed to the patents for such purpose.

The perfluorobromoalkyl ether contrast agents of the invention can be used in a variety of radioimaging applications for medical and veterinary diagnostic purposes. Fields of radiographical use include, for example, gastroenterography, alveolography, bronchography, lymphography, ventriculomyelography, splenography, cholecystography, pancreatography, renal angiography and retrograde urography. With specific reference to renal angiography, the bromoethers of this invention, when employed for instance as emulsions, are unique in that they are not excreted in the urine. Moreover, all the arteries, arterioles, venules and veins of the kidney stand out very clearly. On the other hand, an X-ray made with a conventional iodinated, water-soluble contrast agent provides a solid black, kidney-shaped silhouette because the agent is excreted so rapidly. In the case of lymphangiography, the known water-soluble contrast agents are syrupy and therefore it is very difficult to use a tiny 30 gauge needle for their administration. The perfluorobromoalkyl ether contrast agents, however, go through such a needle very easily and, therefore, facilitate the diagnostic procedure.

When the ether, neat or as an aqueous dispersion, is used as a radiopaque agent, it can be introduced into the animal by various means. Suitable methods include swallowing, injection, and catheterization. The amount used can vary substantially depending in part on the weight of the animal, and the particular region

being X-rayed. The intensity (or amount) of the X-ray irradiation can also influence the amount of ether used. The foregoing factors will routinely be taken into consideration by one skilled in the art to determine the effective amount for the condition being studied.

The ether or ether dispersion should be rendered isotonic or otherwise physiologically acceptable to the blood prior to introduction into the bloodstream by the addition of salts (e.g. as Ringer solution), buffering agents (such as $NaHCO_3$) or other additives commonly employed for such purpose.

The following Example will further illustrate the invention without necessarily limiting the scope thereof, except as set forth in the appended claims.

## Example

Perfluoro-1-bromobutylisopropyl ether, $(CF_3)CFO\ C_4F_8Br$, is prepared in 85% yield by reacting bromine with $(CF_3)_2CFOC_4F_8I$ in the presence of ultraviolet light. The product is purified using a Perkin-Elmer Annular Still and preparative gas chromatography and is characterized by mass spectrometry and infrared spectroscopy. The ether has a boiling point of 113—115°C at 1,0108 Pa (=at 760 torr) and a density of 1.861 g/cc at 24°C. Molecular weight: 446 (calculated and found).

The ether has an LD-50 of over 100 ml/kg as an emulsion containing 10% by volume of the ether and 90% by volume of 5% by weight Pluronic® F68 surfactant in water, and therefore is relatively non-toxic. The Pluronic® F68 surfactant is a polyoxyethylene-polyoxypropylene copolymer having a molecular weight of about 8200. The limits of the toxicity data are as follows:

TABLE 1

| Emulsion dose (ml/kg) | Number alive at time shown 10 min | 1 hour | 7 days |
|---|---|---|---|
| 100 | 3/4 | 3/4 | 3/4 |
| 75 | 4/4 | 4/4 | 4/4 |
| 50 | 4/4 | 4/4 | 4/4 |

To determine emulsion stability, the above-described emulsion of the ether, prepared by ultrasonication, is stored at 2°C in a sealed Pyrex (trademark) tube in a refrigerator. Emulsion stability is determined by measurement of optical density initially and after several days' storage of the samples using a Spectronic 20 apparatus and a suitable size cell. Comparison with emulsions in which perfluorodecalin or perfluorotributyl amine is substituted for the ether indicates that the ether emulsion is reasonably stable. Stability of other perfluorobrominated ether emulsions would be expected to vary.

Transpiration is measured by injecting a mouse with the test emulsion described above and placing the mouse in an all-glass chamber through which oxygen flows at a rate of about 20 cc per minute. After 20 minutes, samples of the oxygen are removed using a microsyringe and analyzed by gas chromatography using an SE-30 column and an electron capture detector. By calibration of the detector with a standard prepared from the perfluoro compound of the emulsion, the rate at which the perfluoro compound is transpired through the skin and lungs of the intact, awake mouse can be calculated. Knowing the amount injected into the mouse and the rate at which it leaves over a period of time, the time at which all of the perfluoro compound would be gone can be calculated.

The results of the foregoing procedure, as compared with essentially the same emulsions except for use of different perfluoro compounds (perfluoro decalin and perfluorooctylbromide), expressed in terms of the rates (µg/min) at which the perfluoro compound leaves the mouse at lapsed times of 5, 10, 20, 30 and 40 weeks, indicate that the perfluoro-1-bromobutylisopropyl ether leaves the body at a rate almost the same as perfluorodecalin (known to transpire quickly) and therefore has a desirable transpiration rate.

Accumulation of the perfluoro-1-bromobutylisopropyl ether in the liver and spleen of mice infused with the above-described either emulsion is determined by direct combustion of liver and spleen homogenates by sodium biphenyl followed by fluoride ion measurement with an electrode. The results are as follows where the infusion dose is 100% cc/kg.

TABLE 2
Wet combustion analysis of liver and spleen

| Days after infusion | Percent of dose remaining | | |
|---|---|---|---|
| | Liver | Average | Spleen |
| 7 | 15.85<br>16.72 | 16.3 | 4.77 |
| 13 | 2.59<br>2.59 | 2.59 | 2.30 |
| 24 | 1.4<br>1.2 | 1.3 | 0.4 |

The data show that the ether went to the liver and spleen, and then left.

In other experiments, tissue examples are taken from mice which are given intravenous doses of the above-described emulsion containing the perfluoro-1-bromobutylisopropyl ether. The emulsion doses are 100, 75 and 50 cc/kg. The mice are sacrificed at 7, 13 and 24 days and samples of liver, spleen and kidney are removed, fixed and mounted for sectioning. Also, a sample of spleen is removed from a mouse at 330 days post-infusion. Some of these tissues are sectioned and examined. During the period when the ether is present in large amounts, microscopic examination reveals little or no mitosis of hepatocytes, but some hypertrophy of nuclei is seen. Binucleated cells are observed and in one case as many as 53% are binucleated. Less than 1% of hepatocyte cytoplasm is occupied by the perfluorobromoalkyl ether. Some infiltration of mononuclear cells is observed and blast-like cells as well as fluorocarbon particles are found in the interstitium. The re-emulsification of the larger fluorocarbon particles into smaller particles, which normally precedes the disappearance, is very near completion. By two weeks post-infusion, the liver is within normal limits with the exception of some phagocytes containing the perfluorobromo compound, and nuclei of hepatocytes are seen somewhat enlarged, but otherwise normal.

A neat sample of the perfluoro-1-bromobutylisopropyl ether is injected into the trachea of an anesthetized rat through a plastic tube having a bevelled end. Serial X-rays are taken and show the ether to be a good contrast agent. Similarly, the above-described emulsion containing the ether is intravenously injected into a mouse. X-rays (50 KVA applied) are taken two days later. The X-rays clearly show the liver and spleen, indicating the the ether is deposited in the reticuloendothelial system. The foregoing results thus demonstrate effective use of the ether as a radiopaque agent.

**Claims**

1. A perfluorobromoalkyl ether of the formula:

$$C_mF_{2m+1}OC_nF_{2n}Br$$

where m and n independently are integers of from 2 to 6, for use as a radiopaque agent.

2. A compound according to Claim 1, wherein the ether is *F*-1-bromobutylisopropyl ether.

3. A compound according to Claim 1 or 2, wherein the radiopaque agent takes the form of an aqueous dispersion thereof.

4. A compound according to Claim 3, wherein the aqueous dispersion contains 50—90% by volume of water, 0.5—10% by weight of a dispersant based on the water content, and 5—20% by volume of the ether.

5. A compound according to any of Claims 1 to 4, for use in a method of radioimaging an internal region of an animal wherein a radiopaque agent is introduced into the region and the region is X-rayed while perfused with the radiopaque agent.

6. An aqueous dispersion comprising a perfluorobromoalkyl ether of the formula:

$$C_mF_{2m+1}OC_nF_{2n}Br$$

where m and n are independently integers of from 2 to 6, and a fluorinated amidoamine oxide of the formula (1):

$$\begin{array}{l} R_fCON\text{—}RQ \\ \quad\quad\;\;| \\ \quad\quad\;\;Y \end{array}$$

wherein $R_f$ is a perfluoroalkyl radical of 1 to 25 carbon atoms or a polyfluoroalkoxyalkyl radical wherein the

alkoxy group contains 3 to 40 carbon atoms of which at least a major portion thereof are perfluorinated and the alkyl group contains 2 to 40 carbon atoms, fluorinated or unfluorinated; Y is a hydrogen or alkyl of 1 to 6 carbon atoms; R is an alkylene radical of the formula:

$$—C_zH_{2z}—$$

wherein z is an integer of 1 to 6; and Q is an aliphatic amine oxide radical of the formula:

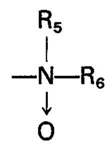

wherein $R_5$ and $R_6$ are each alkyl radicals of 1 to 6 carbon atoms or hydroxy terminated alkyl radicals of 2 to 6 carbon atoms.

**Patentansprüche**

1. Perfluorbromalkylether der Formel

$$C_mF_{2m+1}OC_nF_{2n}Br$$

in der m und n unabhängig voneinander ganze Zahlen von 2 bis 6 bedeuten, zur Verwendung als Strahlenkontrastmittel.

2. Verbindung nach Anspruch 1, wobei der Ether F-1-Brombutylisopropylether ist.

3. Verbindung nach Anspruch 1 oder 2, wobei das Strahlenkontrastmittel in Form einer wäßrigen Dispersion der Verbindung vorliegt.

4. Verbindung nach Anspruch 3, wobei die wäßrige Dispersion 50—90 Vol.-% Wasser, 0,5—10 Gew.-%, bezogen auf den Wassergehalt, eines Dispergiermittels und 5—20 Vol.-% des Ethers enthält.

5. Verbindung nach einem der Ansprüche 1 bis 4 zur Verwendung in einem Verfahren zur Röntgenabbildung eines inneren Bereiches eines Tieres, wobei ein Röntgenkontrastmittel in den Bereich eingeführt wird und der Bereich geröntgt wird, während er mit dem Röntgenkontrastmittel durchspült wird.

6. Wäßrige Dispersion, enthaltend einen Perfluorbromoalkylether der Formel

$$C_mF_{2m+1}OC_nF_{2n}Br$$

in der m und n unabhängig voneinander ganze Zahlen von 2 bis 6 sind, sowie ein fluoriertes Amidoaminoxid der Formel (1):

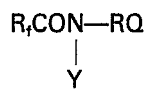

worin $R_f$ einen Perfluoralkylrest mit 1 bis 25 Kohlenstoffatomen oder einen Perfluoralkoxyalkylrest bedeutet, worin die Alkoxygruppe 3 bis 40 Kohlenstoffatome aufweist, von dem mindestens der überwiegende Anteil perfluoriert ist, und die Alkylgruppe 2 bis 40 Kohlenstoffatome enthält, die fluoriert oder nicht fluoriert sind, Y für Wasserstoff oder eine Alkylgruppe mit 1 bis 6 Kohlenstoffatomen steht, R einen Alkylenrest der Formel

$$—C_zH_{2z}—$$

bedeutet, worin z eine ganze Zahl von 1 bis 6 ist; und Q einen aliphatischen Aminoxidrest der Formel:

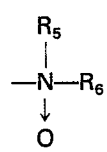

bedeutet, worin $R_5$ und $R_6$ jeweils Alkylreste mit 1 bis 6 Kohlenstoffatomen oder Alkylreste mit 2 bis 6 Kohlenstoffatomen mit endständigen Hydroxygruppen bedeuten.

**Revendications**

1. Ethers perfluorobromoa-alkylique de formule:

$$C_mF_{2m+1}OC_nF_{2n}Br$$

dans laquelle m et n, pris indépendamment, sont des nombres entiers de 2 à 6, pour l'utilisation comme agent opaque aux rayons.

2. Composé selon la revendication 1, dans lequel l'éther est le *F*-bromo-1 butyloxy propane.

3. Composé selon une des revendications 1 ou 2, dans lequel l'agent opaque aux rayons prend la forme d'une dispersion aqueuse.

4. Composé selon la revendication 3, dans lequel la dispersion aqueuse renferme de 50 à 90 % en volume d'eau, 0,5 à 10 % en poids rapporté à la teneur en eau d'un agent de dispersion, et 5 à 20% en volume de l'éther.

5. Composé selon une des revendications 1 à 4, desinté à être utilisé dans un procédé de représentation radiologique d'une région interne d'un animal, au cours duquel on introduit dans la-dite région, soumise à un rayonnement X, une perfusion de l'agent opaque aux rayons.

6. Dispersion aqueuse comprenant un éther perfluorobromoalkylique de formule:

$$C_mF_{2m+1}OC_nF_{2n}Br$$

dans laquelle m et n sont des nombres entiers, indépendants, de 2 à 6, et un oxyde d'amidoamine fluoré de formule (1):

$$\begin{array}{c} R_fCON\text{—}RQ \\ | \\ Y \end{array}$$

dans laquelle $R_f$ est un radical perfluoroalkyle de 1 à 25 atomes de carbone, ou un radical polyfluoroalkoxyalkyle dans lequel le groupement alkoxy renferme 3 à 40 atomes de carbone dont au moins une partie majeure est perfluorée et le groupement alkyle renferme 2 à 40 atomes de carbone, fluorés ou non fluorés; Y est un hydrogène ou un alkyle de 1 à 6 atomes de carbone; R est un radical alkylène de formule:

$$\text{—}C_zH_{2z}\text{—}$$

dans laquelle z est un nombre entier de 1 à 6; et Q est un radical oxyde d'amine aliphatique de formule:

$$\begin{array}{l} R^5 \\ | \\ N\text{—}R^6 \\ \downarrow \\ O \end{array}$$

dans laquelle $R^5$ et $R^6$ sont chacun un radical alkyle de 1 à 6 atomes de carbone, ou un radical alkyle de 2 à 6 atomes de carbone terminé par un hydroxyle.